(19) Europäisches Patentamt
European Patent Office
Office européen des brevets

(11) **EP 2 854 497 B1**

(12) # EUROPÄISCHE PATENTSCHRIFT

(45) Veröffentlichungstag und Bekanntmachung des Hinweises auf die Patenterteilung:
**01.03.2017  Patentblatt 2017/09**

(21) Anmeldenummer: **13726227.5**

(22) Anmeldetag: **03.06.2013**

(51) Int Cl.:
**A01C 1/08** (2006.01)    **A61L 2/08** (2006.01)

(86) Internationale Anmeldenummer:
**PCT/EP2013/061338**

(87) Internationale Veröffentlichungsnummer:
**WO 2013/182500 (12.12.2013 Gazette 2013/50)**

(54) **VERFAHREN UND VORRICHTUNG ZUR DESINFEKTION RIESELFÄHIGER PRODUKTE, VORZUGSWEISE SAATGUT, MIT BESCHLEUNIGTEN ELEKTRONEN**

METHOD AND DEVICE FOR DISINFECTION GRANULAR PRODUCTS, SUCH AS SEED, WITH ACCELERATED ELECTRONS.

PROCÉDÉ ET DISPOSITIF POUR DÉSINFECTER DES PRODUITS GRANULEUX, COMME SEMENCE, AVEC ÉLECTRONS ACCÉLÉRÉS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.06.2012   DE 102012209434**

(43) Veröffentlichungstag der Anmeldung:
**08.04.2015   Patentblatt 2015/15**

(73) Patentinhaber: **Evonta-Service GmbH**
**01454 Radeberg (DE)**

(72) Erfinder:
• **KOTTE, Mathias**
**01847 Lohmen (DE)**
• **RÖDER, Olaf, Dr.**
**01326 Dresden (DE)**

(74) Vertreter: **Kailuweit & Uhlemann Patentanwälte Partnerschaft mbB**
**Bamberger Straße 49**
**01187 Dresden (DE)**

(56) Entgegenhaltungen:
**EP-A1- 1 080 623     DE-A1-102006 020 483**
**US-A- 5 801 387**

EP 2 854 497 B1

**Beschreibung**

Die Erfindung betrifft ein Verfahren und eine Vorrichtung zur Desinfektion rieselfähiger Produkte mit beschleunigten Elektronen. Unter rieselfähig sind alle körnigen, auch als Schüttgut, Pulver oder Granulat bezeichneten, Produkte zu verstehen, die in einem kontinuierlichen Stoffstrom transportiert und vereinzelt werden können. Das bevorzugte Anwendungsgebiet der Erfindung ist die Behandlung pflanzlicher Samen bzw. Saatgut durch Abtötung und Inaktivierung der Mikroorganismen und tierischer Schädlinge, die überwiegend auf der Oberfläche sowie in und unter der Samenschale der Produkte siedeln oder separat mit dem Produktstrom gefördert werden. Bekannt sind verschiedene Verfahren und die entsprechenden Einrichtungen zur Behandlung von Schüttgut mit beschleunigten Elektronen. So sind Verfahren beschrieben, bei denen Schüttgut in eine Vakuumkammer eingeschleust und im Fallen mit Elektronen behandelt wird (DD 291 704; D D 291 705; D D 291 677). Durch Anordnung einer oder mehrerer Axial-Elektronenbeschleuniger mit je einem über die Breite des Schüttgutvorhanges im Vakuum gescannten Strahl soll eine allseitige und gleichmäßige Beaufschlagung mit Elektronen erreicht werden. Bei der Prozessführung im Vakuum werden alle Teilchen, unabhängig von ihrem Luftwiderstand, nach einer bestimmten Fallhöhe durch die Erdgravitation auf die gleiche Geschwindigkeit beschleunigt, da keine Luftreibung vorliegt. Bei definierter Geschwindigkeit hat jedes Partikel die gleiche Expositionszeit im Elektronenstrahl. Alle Partikel erhalten so die gleiche Elektronendosis.

Ein wesentlicher Mangel dieses Verfahrens besteht darin, dass die Erzeugung und Aufrechterhaltung des Vakuums in der Behandlungskammer einen sehr hohen Evakuierungsaufwand erfordert. Dies ist dadurch bedingt, dass mit dem Schüttgut kontinuierlich Luft und Wasserdampf in die Behandlungskammer eingetragen werden. Ein weiterer Mangel ist die zur Druckabstufung erforderliche Anordnung mehrerer in Reihe geschalteter Produktschleusen am Ein- und Auslauf der Vakuumkammer, was eine enorme Bauhöhe der Gesamtanlage zur Folge hat. Die hohen Kosten für die Erzeugung des Vakuums, die erforderlichen Produktschleusen sowie das Erzeugen und Scannen des Elektronenstrahles begrenzen die Anwendung des Verfahrens auf hochpreisige Spezialprodukte.

Es sind außerdem Einrichtungen bekannt, die durch Pumpen evakuierte Bandstrahler zur Erzeugung des Elektronenstrahles nutzen. Die Elektronen gelangen über ein Elektronenaustrittsfenster, auch Strahlaustrittsfenster genannt, aus dem evakuierten Strahlerzeugungsraum der Bandstrahler in eine unter Atmosphärendruck stehende Prozesskammer (D 4 4 34 767; EP 07 05 531). Das Schüttgut fällt innerhalb der Prozesskammer im freien Fall in einer Gasatmosphäre, in der Regel Luft, durch das Elektronenfeld. Zur Kühlung des Elektronenaustrittsfensters sind Düsen angeordnet, die am Elektronenaustrittsfenster einen Gasstrom erzeugen. Durch diesen Kühlgasstrom soll auch der Staub vom Elektronenaustrittsfenster ferngehalten werden.

Ein wesentlicher Mangel derartiger Einrichtungen mit Prozessführung unter Gasatmosphäre besteht darin, dass die Partikel des Schüttgutes im freien Fall durch Einwirkung der Erdgravitation stetig beschleunigt werden, durch das Gas in der Behandlungskammer jedoch ein Strömungswiderstand wirkt, der die Partikel in Abhängigkeit von ihrer Größe, Dichte, Form und Oberflächenbeschaffenheit unterschiedlich abbremst. Dadurch fallen Partikel mit einem großen Verhältnis von Oberfläche zu Volumen, wie z.B. Getreidespelzen, wesentlich langsamer als kugelförmige Partikel mit kleiner Oberfläche wie z.B. ein Getreidekorn. Die daraus resultierende oft erhebliche Geschwindigkeitsdifferenz verschiedener Partikel des Saatgutstromes führt zu sehr unterschiedlichen Einwirkzeiten der Elektronen und damit zu einer breiten Streuung der Elektronendosis im Schüttgutstrom. Dies hat zur Folge, dass Partikel, die eine hohe Elektronendosis erhalten, geschädigt werden können, während bei Partikeln mit geringer Elektronendosis die zur Abtötung der Mikroorganismen und Insekten erforderliche Letaldosis nicht erreicht wird. Durch überlebende Organismen kann nach der Behandlung eine Reinfektion der übrigen Partikel unter Vermehrung der Organismen einsetzen, die zu einem unerwünschten Befall und damit zum Qualitätsverlust des gesamten Produktvolumens führt.

Bei den bekannten Einrichtungen werden Elektronenbeschleuniger eingesetzt, deren Anordnung gegenüberliegend erfolgt. Der Nachteil einer gegenüberliegenden Anordnung besteht darin, dass die beschleunigten Elektronen in Abhängigkeit ihrer Energie das Elektronenaustrittsfenster des jeweils gegenüberliegenden Elektronenbeschleunigers erreichen und durch überhöhten Energieeintrag thermisch schädigen können. Eine derartige Anordnung begrenzt die einsetzbare Elektronenenergie und den Elektronenstrom und damit die erzielbare Produktivität und Wirksamkeit der Einrichtung.

Werden die Elektronenbeschleuniger zur Vermeidung dieses Nachteiles in der Höhe versetzt angeordnet, hat dies eine unterschiedliche Distanz zur darüber liegenden Produktzuführung zur Folge. Da die Partikel im freien Fall einer steten Beschleunigung unterliegen, ist ihre Geschwindigkeit beim Passieren des oberen Elektronenaustrittsfensters geringer als im Bereich des unten liegenden, was zur Übertragung unterschiedlicher Energiedosen führt. Bei bekannten Einrichtungen (DE 44 34 767; EP 07 05 531) besteht zusätzlich das Problem der Geschwindigkeitsunterschiede unterschiedlicher Partikelformen durch den Luftwiderstand. Durch Kontakt der Partikel mit der angrenzenden Wand kann es außerdem zu unkontrollierten Quer- und Pendelbewegungen einzelner Partikel kommen. Aus den genannten Nachteilen resultiert, dass Partikel auf einer Seite die zweifache Dosis erhalten können, wobei auf der dem Elektronenstrahl abgewandten Seite nur eine verminderte Dosis übertragen wird.

Ein weiterer Nachteil der beschriebenen Lösung besteht darin, dass der Gasstrom zur Kühlung des Elektronenaustrittsfensters aufgrund des geschwindigkeitsbedingten dynamischen Druckes des Gasstromes zu einer Absenkung des statischen Druckes zwischen Elektronenaustrittsfenster und Schüttgutstrom führt. Der daraus resultierende Druckausgleich hat eine Gasströmung von der Behandlungszone in Richtung Elektronenaustrittsfenster zur Folge, der leichte Partikel wie z.B. Staub oder Spelzen mit sich reißt. Partikel, die größer als die Maschenweite des Schutzgitters zwischen Produktstrom und Elektronenaustrittsfenster sind, bleiben dort hängen, setzen das Gitter sukzessive zu und führen zu einer unerwünschten Verringerung der Elektronentransparenz des Gitters. Kleinere Partikel können durch die Maschen des Schutzgitters hindurch auf das Elektronenaustrittsfenster gelangen und führen durch abrasive Wirkung zur Verringerung der Lebensdauer der empfindlichen Folie des Elektronenaustrittsfensters.

Ein zusätzlicher Mangel besteht darin, dass der eingesetzte Bandstrahler zur Aufrechterhaltung des Hochvakuums in der Beschleunigerkammer permanent evakuiert werden muss. Die dafür erforderlichen Hochvakuumpumpen bedingen einen hohen apparativen und wartungstechnischen Aufwand und schränken das Verfahren in seiner Wirtschaftlichkeit ein.

Es sind außerdem Verfahren und Vorrichtungen zur Behandlung von Schüttgut bekannt, bei denen die erforderliche Energiedosis durch mehrmaliges Durchlaufen des Elektronenfeldes erzielt wird. Dadurch soll die Homogenität der Behandlung erhöht werden (EP 10 80 623). Wesentlicher Nachteil des Verfahrens ist, dass die in der Behandlungskammer durch Elektroneneinwirkung erzeugte Röntgenstrahlung eine Röntgendosis im Volumen jedes Schüttgutpartikels erzeugt, die sich bei jedem Durchlauf addiert. Bei der Anwendung an lebenden Produkten, wie beispielsweise Saatgut, wirkt diese erhöhte Röntgendosis auf den Embryo im Korninneren und kann zu unerwünschten genetischen Veränderungen des Saatgutes und damit zu erheblichen Qualitätsverlusten führen.

Der Erfindung liegt die Aufgabe zugrunde, ein Verfahren und eine zugehörige Vorrichtung zu schaffen, die es ermöglichen, rieselfähige Produkte unabhängig von der Größenverteilung der Partikel sowie deren Dichte, Form und Oberflächenbeschaffenheit ohne Evakuierung der Behandlungskammer mit großer Homogenität des Energieeintrages auf die Einzelpartikel und auf deren gesamten Oberfläche so zu behandeln, dass die zur sicheren Abtötung der Mikroorganismen und Insekten erforderliche Elektronendosis an jedem Oberflächenelement erreicht und eine Qualitätsminderung oder genetische Veränderung des Produktes durch zu hohe Elektronendosis oder/und zu hohe Röntgendosis jedoch vermieden wird. Insbesondere ist es Ziel, durch die Behandlung mit beschleunigten niederenergetischen Elektronen schädliche Mikroorganismen und Insekten abzutöten, die an der Oberfläche und in einer Randschicht der Partikel bis zu einer Tiefe von ca. 500 $\mu$m siedeln.

Erfindungsgemäß wird die Aufgabe durch das Verfahren gemäß Patentanspruch 1 gelöst. Gegenstand des Anspruches 9 ist eine zur Durchführung des Verfahrens geeignete Einrichtung. Besonders vorteilhafte Ausgestaltungen sind in den Ansprüchen 2 bis 8 und 10 bis 22 beschrieben.

Nach dem erfindungsgemäßen Verfahren wird der Strom des rieselfähigen Produktes zunächst mittels einer Fördereinrichtung in eine Behandlungskammer transportiert, wobei bevorzugt bereits eine mechanische Vorvereinzelung der Partikel erfolgt. Das hat den Vorteil, dass auch bei großen Produktdurchsätzen die Partikel hinreichend separiert und mit vernachlässigbarer Überdeckung der Behandlungskammer zugeführt werden. Die Behandlungskammer sowie die Bereiche der Produktzuführung und -abführung sind gegen den Austritt von Röntgenstrahlung geschirmt. Die Produktzuführung und -abführung erfolgt durch geeignete Fördereinrichtungen, wie Vibrationsförderrinnen, Hochgeschwindigkeitsbänder oder Zellenradschleusen. In Kombination mit einer angepassten Röntgenschutzverkleidung ermöglicht dies einen kontinuierlichen Stoffstrom bei gleichzeitiger Verhinderung des Austritts von Röntgenstrahlung. Beim Eintritt der Partikel in den, in der Regel vertikal nach unten, gerichteten Produktführungskanal, erfolgt durch Einwirkung der Schwerkraft eine kontinuierliche Beschleunigung der Partikel. Dadurch nimmt die Translationsgeschwindigkeit v der Partikel nach der Gesetzmäßigkeit

$$v = \sqrt{2 * g * h} \qquad\qquad (1)$$

stetig zu, wobei g die Erdbeschleunigung und h der zurückgelegte Fallweg der Partikel ist. Der Produktführungskanal ist mit einer Gasfördereinrichtung, vorzugsweise einem Gasgebläse, verbunden, die einen konstanten Gasvolumenstrom innerhalb des Produktführungskanals in Fallrichtung des Produktstromes erzeugt. Es bildet sich ein vertikal nach unten, in Transportrichtung, im Gasvolumenstrom geführter Partikelstrom aus. Durch die Gasfördereinrichtung wird entweder oberhalb des Produktführungskanals ein Überdruck oder am unteren Ende des Produktführungskanals ein Unterdruck erzeugt, der die Gasbewegung in Fallrichtung bewirkt. Der Über- bzw. Unterdruck ist nicht so groß, das spezielle Schleusungs- oder Druckfestigkeitsmaßnahmen notwendig sind, bedeutet jedoch eine deutliche Abweichung vom Atmosphärendruck. Der Druckaufbau ist insbesondere möglich, weil die Zu- oder Abführvorrichtungen für das Produkt einen erheblichen Strömungswiderstand gegen zu- oder abströmende Luft darstellen.

In einer bevorzugten Ausführungsform wird Luft am oberen Ende des Produktführungskanals eingesaugt und in diesen eingeleitet. Am unteren Ende wird die Luft ausgestoßen. Dazu befinden sich am unteren und/oder oberen Ende des Produktführungskanals Gasfördereinrichtungen. Bei einer Gasfördereinrichtung, die am oberen Ende des Produktführungskanals angeordnet ist, herrscht im Inneren der Behandlungskammer ein Überdruck. Ist die Gasfördereinrichtung am unteren Ende des Produktführungskanals angeordnet, so wird das Gas aus diesem angesaugt und es herrscht Unterdruck. Ist am unteren und oberen Ende des Produktführungskanals eine Gasfördereinrichtung vorgesehen, so herrscht im oberen Teil des Produktführungskanals Überdruck und im unteren Unterdruck. An Stellen, an denen Gas bzw. Luft aus dem Produktführungskanal austritt, sind die zugehörigen Öffnungen vorteilhaft mit Vorrichtungen versehen, die einen Austritt von Produkt verhindern. Dies sind vorzugsweise Gitter, Siebe, Gaze oder Lochbleche, bevorzugt in Verbindung mit zugehörigen Reinigungseinrichtungen. In dem nach außen geführten Gasstrom ist in einer bevorzugten Ausführungsform vor dem Ausblasen in die Umwelt ein Ozonkatalysator vorgeschaltet, der das aus Sauerstoff in der Behandlungszone entstehende Ozon reduziert und bevorzugt auf ein akzeptables Maß abbaut.

Weiterhin bevorzugt wird der Gasstrom von der Gasfördereinrichtung im Kreislauf geführt, indem er am unteren Ende des Produktführungskanals wieder zu dessen oberen Ende umgelenkt wird. Zwischen der Abführung des Gasstromes am unteren Ende des Produktführungskanals und der Zuführung am oberen Ende, ist bevorzugt die Gasfördereinrichtung angeordnet. Eine besonders bevorzugte Ausführungsform sieht eine Reinigungseinrichtung für den umgelenkten Gasstrom vor. Diese Reinigungseinrichtung entfernt Staub aus dem Gasstrom und verhindert dessen Anreicherung im umlaufenden Gasstrom. Die Reinigungseinrichtung ist dabei bevorzugt als Filter (Schlauchfilter), Zyklon oder eine ähnliche Vorrichtung nach dem Stand der Technik ausgebildet. Bevorzugt wird die Abführung des Gasstromes durch ein Schutzgitter, das bevorzugt automatisch oder manuell reinigbar ist, gegen das Einsaugen bzw. Ausblasen größerer Partikel verschlossen. Verluste an Gas, die aus dem Austritt über die Produktzu- oder abführung resultieren, werden je nach Art des Gases im Gasstrom ggf. durch weitere Gasfördereinrichtungen, durch eine ergänzende Zufuhr aus Gastanks oder durch Ansaugvorrichtungen aus der Atmosphäre, ausgeglichen.

Eine weitere bevorzugte Ausführungsform sieht mehrere Gasfördereinrichtungen vor, die am unteren und/oder oberen Ende des Produktführungskanals angeordnet sind.

Der Gasstrom wird bevorzugt von Luft, Stickstoff oder Kohlendioxid gebildet. Bevorzugt sind inerte Gase, die keine unbeabsichtigten chemischen Reaktionen mit den Produkten eingehen.

Eine besonders bevorzugte Ausführungsform sieht den Zusatz von Behandlungsgasen vor, die beabsichtigte chemische oder biologische Reaktionen in oder an den Partikeln hervorrufen.

Der Querschnitt des Produktführungskanals nimmt nach unten, in Fallrichtung des Produktstromes kontinuierlich ab. Durch den so gestalteten Querschnitt des Produktführungskanals wird der Gasstrom annähernd in gleichem Maße wie der Produktstrom beschleunigt, wodurch die Geschwindigkeit des umgebenden Gases an jedem Punkt mit der Geschwindigkeit der im Fall beschleunigten Partikel weitgehend übereinstimmt. Die Führung des Produktstromes erfolgt mittels eines beschleunigten Gasstromes, dessen Bewegung in Betrag und Richtung der beschleunigten Bewegung entspricht, die die in ihm fallenden Partikel aufgrund der Erdbeschleunigung ausführen. Luftreibung, wie sie bei bekannten Lösungen an Gasatmosphäre auftritt, ist somit ausgeschlossen oder zumindest vernachlässigbar und alle Partikel eines Schüttgutstromes haben näherungsweise identische Geschwindigkeit. Dies hat den Vorteil, dass rieselfähige Produkte unabhängig von der Zusammensetzung sowie der Art, Form und Größe der Partikel mit definierter Geschwindigkeit durch eine Behandlungszone der Elektronen geführt werden und auf diese Weise auf alle Partikel eine homogene Elektronendosis appliziert wird.

Nach oder unmittelbar bei Verlassen der Fördereinrichtung zur Produktzuführung bzw. beim Eintritt in den Produktführungskanal, erhalten die einzelnen Partikel in einer bevorzugten Ausführungsform einen Impuls, der zu einer Rotationsbewegung führt. Die Rotationsachse der Partikel verläuft dabei bevorzugt parallel zur längsten Ausdehnung der Elektronenbeschleuniger. Diese Rotationsbewegung behalten die Partikel während des Aufenthaltes in einem Produktführungskanal und während der Einwirkung der Elektronen im Bereich der Behandlungszone bei. Insbesondere bei Verwendung nur eines Elektronenbeschleunigers bringt dies den Vorteil mit sich, dass die aus einer Vorzugsrichtung auf die Partikel einwirkenden Elektronen die gesamte Oberfläche auf dem Umfang der Partikel erreichen können und eine weitere Verbesserung der Homogenität der Elektronendosis erzielt wird, ohne dass aufwändige Maßnahmen zur Reflektion oder Umlenkung der beschleunigten Elektronen erforderlich sind. Auch die Notwendigkeit einer Mehrfachbehandlung entfällt, da die erforderliche Homogenität und Höhe der Energiedosis in einem Durchlauf erzielt werden. Es wird somit eine im Vergleich zu bekannten Verfahren erhöhte Produktivität erzielt und die beschriebenen Risiken eines Qualitätsverlustes des Produktes durch zu hohe Röntgendosis, wie sie bei der Mehrfachbehandlung auftreten können, sind ausgeschlossen.

Diese Rotationsbewegung wird z. B. über das Abrollen auf einer Schräge, oder eine bürsten- oder walzenartige Vorrichtung, die im Moment des Verlassens der Fördereinrichtung eine Rotation auf die Teilchen überträgt, realisiert.

Der Querschnitt des Produktführungskanals ist in einer bevorzugten Ausführungsform rechteckig. Der Querschnitt weist dabei im Bereich der Wirkzone der Elektronen zwei gegenüberliegende längere und zwei gegenüberliegende kürzere Seiten auf. Die Verringerung des Querschnittes wird erreicht, indem die beiden gegenüberliegenden längeren Seiten sich mit zunehmendem Fallweg der Partikel einander annähern. Die beiden kurzen Seiten verlaufen vorzugsweise parallel. Der Produktstrom rieselt als transparenter "Vorhang", vorzugsweise mittig, zwischen den längeren Seiten des Querschnitts, bevorzugt über deren gesamte Länge, nach unten. Transparenter Vorhang bedeutet in diesem Zusammenhang, dass die gegenseitige Bedeckung der fallenden Partikel, gesehen von den längeren Seiten des Querschnitts, möglichst gering ist, während die Partikel vorteilhaft seitlich einen möglichst geringen Abstand zueinander aufweisen.

Die Spaltbreite s zwischen den zwei gegenüberliegenden längeren Seiten des Produktführungskanals nimmt bei dieser Ausführungsform in Fallrichtung des Produktstromes kontinuierlich ab und ist bevorzugt so gestaltet, dass sie dem Zusammenhang

$$s = k * \frac{1}{\sqrt{h}} \qquad\qquad (2)$$

folgt, wobei h der zurückgelegte Fallweg der Partikel ist und k eine Konstante, die zwischen 170 $mm^{3/2}$ und 250 $mm^{3/2}$ beträgt.

Eine weitere bevorzugte Ausführungsform sieht einen ringförmig gestalteten Spalt vor, der sich ebenfalls mit der zurückgelegten Fallhöhe verengt. Die Produktzuführung erfolgt hierbei oberhalb einer Mittelsäule, die von dem Ringspalt umgeben ist. Weiterhin bevorzugt erfolgt die Produktzuführung durch die Mittelsäule, bspw. mittels eines Schneckenförderers. In einer bevorzugten Ausführungsform ist die Schnecke mit einer elastischen Kante oder mit elastischen Borsten ausgestattet. Am oberen Ende der Mittelsäule tritt das Produkt aus und stürzt über den Rand in den Ringspalt. In einer bevorzugten Ausführungsform wird auch hier den Partikeln eine Rotationsbewegung aufgeprägt, die sie im Fall beibehalten. Dies erfolgt bspw. über eine Zulaufstrecke am Rand der Mittelsäule, über die die Partikel hinab rollen und dabei in Rotationsbewegung geraten.

Nach einer gewissen Fallhöhe (bevorzugt zwischen 20% und 80%, besonders bevorzugt zwischen 30% und 70% und ganz besonders bevorzugt zwischen 40% und 60% der Gesamtfallhöhe) erreichen die Partikel die Behandlungszone. Hier sind ein oder mehrere Elektronenbeschleuniger seitlich an dem Produktführungskanal angeordnet. Sie sind in ihrer Form dem Produktführungskanal angepasst und emittieren Elektronen vorzugsweise über die gesamte Breite des Produktstromes. Insbesondere werden bei einem rechteckigen Produktführungskanal ein oder mehrere linear ausgedehnte Elektronenbeschleuniger eingesetzt, die sich mit der Längsachse parallel zu den längeren Seiten des Querschnitts und senkrecht zur Fallrichtung erstrecken. Werden in einer Einrichtung zwei oder mehr Elektronenbeschleuniger eingesetzt, so kann der erforderliche Elektronenstrom vorteilhaft exakt an die Geschwindigkeit der Partikel und des Gasstromes bei entsprechender Fallhöhe h angepasst werden, wodurch an jedem Ort eine konstante Dosis übertragen wird. Die Energie der Elektronen liegt bevorzugt in einem Bereich von 50keV bis 500 keV, besonders bevorzugt im Bereich von 70keV bis 300 keV und ganz besonders bevorzugt im Bereich von 80 keV bis 200 keV.

Unkontrollierte Quer- und Pendelbewegungen einzelner Partikel, wie sie bei bekannten Verfahren und Einrichtungen auftreten, werden bevorzugt durch Verminderung der Reibung am Produktführungskanalunter Einsatz strömungstechnischer Mittel vermindert. Das erfindungsgemäße Verfahren ermöglicht es so, die entscheidenden Vorteile einer kostengünstigen Prozessführung unter den Bedingungen unterhalb oder oberhalb des Atmosphärendruckes und bei homogener Übertragung der Elektronendosis auf beliebige Partikelgrößen und -Formen im Produktstrom mit einer verbesserten Wirksamkeit der Bekämpfung von Mikroorganismen und Insekten zu kombinieren. Da der Prozess nicht im Vakuum geführt wird, entfällt der bisher erhebliche vakuumtechnische Aufwand.

Der bzw. die Elektronenbeschleuniger arbeiten typischerweise unter Vakuumbedingungen. Eine bevorzugte Ausführungsform sieht vor, hier gekapselte Elektronenbeschleuniger vorzusehen, bei denen der Elektronenstrom über Fenster austritt, die aus einem für Elektronen besonders gut durchlässigen Material besteht, das jedoch die Vakuumbedingungen im jeweiligen Elektronenbeschleuniger bewahrt. Durch eine bevorzugte besondere Kapselung des Elektronenbeschleunigers mittels Löt- und oder Schweißverbindungen entfallen üblicherweise erforderliche Hochvakuumpumpen für die permanente Aufrechterhaltung des Vakuums innerhalb der Beschleunigerkammer. So kann vorteilhaft auf Vakuumpumpen komplett verzichtet werden. Bei Einsatz eines oder mehrerer hermetisch gekapselter Elektronenbeschleuniger erhöht dies die Wirtschaftlichkeit des Verfahrens im Vergleich zu bekannten Lösungen erheblich.

Das erfindungsgemäße Verfahren beinhaltet die beschriebene aktive Gasführung innerhalb des Produktführungskanals. Durch die Geschwindigkeit dieser kontinuierlich beschleunigten Gasströmung und den daraus resultierenden erhöhten dynamischen Druck wird der statische Druck im Produktführungskanal abgesenkt. Der Produktführungskanal ist im Bereich des Elektronenaustrittsfensters für den Durchtritt der Elektronen in die Behandlungszone des Produktführungskanals geöffnet. Zum Schutz vor dem Austritt von Produkt aus dem Produktführungskanal sind in einer bevorzugten Ausführungsform die Öffnung bzw. die Öffnungen durch eine flächige Perforation der Kanalwand realisiert oder mit einer engmaschigen Gaze bzw. einem Gitter abgedeckt. Durch diese Öffnungen kann in einer bevorzugten Ausführungsform Gas, vorzugsweise Luft, in den Produktführungskanal zuströmen, da der verringerte statische Druck im Produktführungskanal zu einer Bypass-Gasströmung führt, die vom Elektronenaustrittsfenster in Richtung Produktführungskanal gerichtet ist. Das nachströmende Gas wird über das Elektronenaustrittsfenster geführt, wodurch dieses konvektiv gekühlt wird. Zur Einstellung der Druckverhältnisse innerhalb des Produktführungskanals kann in einer Ausführungsform das nachströmende Gas mittels eines separaten Hilfsgebläses gestützt werden. Ein Vorteil dieser Kühlung besteht darin, dass die in den Produktführungskanal gerichtete Bypass-Gasströmung den perforierten bzw. mittels Gitter abgedeckten Bereich, durch den die beschleunigten Elektronen in die Behandlungszone geführt werden, von Ablagerungen frei hält. Dadurch entfallen in dieser Ausführungsform auch die sonst üblichen zusätzlichen Mittel der Kühlgaserzeugung mittels großer Gebläse, was weitere wirtschaftliche Vorteile bietet.

Durch die aktive Gasführung im Produktführungskanal und durch die Bypass-Gasströmung in den Kanal wird eine Verringerung der Reibung zwischen den Partikeln des Produktstromes und der Wandung in diesem Bereich bewirkt. Dies kann auch zur gezielten Reibungsminderung in anderen Teilbereichen des Produktführungskanals eingesetzt werden. Dadurch sind Quer- und Pendelbewegungen einzelner Partikel minimiert, was zu einer zusätzlichen Verbesserung der Dosishomogenität führt. Bevorzugt wird die Bypass-Gasströmung als Teilstrom von dem im Kreis geführten Gasstrom, der von der Gasfördereinrichtung bewegt wird, abgezweigt. Die drucktechnische Auslegung sichert, dass der Bypass-Gasstrom in der notwendigen Menge eintritt. Eine zugehörige Regelung erfolgt vorteilhaft mittels bekannter Vorrichtungen (Klappen, Schieber etc.).

Bevorzugt ist im Produktführungskanal mindestens ein Druckmesser angeordnet. Besonders bevorzugt sind mehrere Druckmesser über die Höhe des Produktführungskanals verteilt. In einer bevorzugten Ausführungsform wird die gesamte Anlage mittels einer elektronischen Datenverarbeitungsvorrichtung gesteuert. Die Drucksensoren sowie die weiteren Sensoren (bspw. Sensoren für Elektronenstromdichte und Dosismessgeräte für Röntgenstrahlung, Temperatursensoren an den Elektronenbeschleunigern, Sensoren zur Messung des Produktstromes etc.) übermitteln die erfassten Daten vorteilhaft ebenfalls an die Datenverarbeitungsvorrichtung. Die Übermittlung kann drahtlos oder drahtgebunden erfolgen. Die Datenverarbeitungseinrichtung steuert dann vorteilhaft sowohl den Produktstrom mittels der Fördereinrichtungen als auch den mindestens einen Elektronenbeschleuniger und die eine oder mehrere Gasfördereinrichtungen.

Die Einstellung der Verfahrensparameter (Elektronenstrom, Beschleunigungsspannung, Gasart, Gasdruck) erfolgt bei Saatgut anhand morphologischer Merkmale (Samenschalendicke und -dichte, Lage des Embryos, Samenart). Mittels der bekannten Berechnungsverfahren zur Bestimmung der Eindringtiefe beschleunigter Elektronen in Materie lassen sich so rechnerisch die notwendigen Prozessbedingungen ermitteln. Dies erfolgt vorzugsweise in der Datenverarbeitungseinrichtung oder im Vorhinein, wobei die notwendigen Angaben dann vor Prozessbeginn in die Datenverarbeitungseinrichtung eingegeben werden.

Eine weiterhin bevorzugte Ausführungsform sieht vor, zwei oder mehr Elektronenbeschleuniger anzuordnen. Um eine gegenseitige thermische Beeinflussung von Elektronenbeschleunigern, die sich bevorzugt gegenüberstehen, auszuschließen, werden diese in der Höhe versetzt angeordnet und die Leistung an die jeweilige Geschwindigkeit des Produktstromes angepasst. Eine weitere vorteilhafte Variante ist die gegenüberliegende Anordnung der Elektronenbeschleuniger unter Verdrehung um die horizontale Längsachse im Winkelbereich von 5° bis 45°, so dass der mittlere Geschwindigkeitsvektor der Elektronen zum Geschwindigkeitsvektor des Partikelstromes um diesen Winkelbetrag von der Orthogonalen abweicht. Diese Anordnung kann auch bei versetzten Elektronenbeschleunigern gewählt werden und ermöglicht hohe Elektronenströme zur Erzielung hoher Energiedosen bei verbesserter Gleichmäßigkeit des Energieeintrages auf die Partikel des Produktstromes.

In einer weiteren bevorzugten Ausführungsform sind Mittel für den Brand- und Explosionsschutz vorgesehen. Eingesetzt werden insbesondere Funkendetektoren, die nicht auf die Wellenlänge des Tageslichts und des durch Elektronen erzeugten (leuchtenden) Gasplasmas in der Wirkzone ansprechen.

Die erfindungsgemäße Vorrichtung ist neben dem Einsatz zur Desinfektion bzw. Sterilisation von Saatgut auch für weitere rieselfähige Produkte geeignet. Dies sind beispielsweise pharmazeutische Produkte, wie Tabletten, Granulate, Kapseln, sowie alle Arten kontaminierter rieselfähiger Massen wie z. B. Erde, Kunststoffabfälle (geschreddert), Rezyklate etc.

An einem Ausführungsbeispiel wird die Erfindung näher erläutert. In den zugehörigen Zeichnungen zeigen:

Fig. 1: einen Schnitt durch eine Einrichtung zur Desinfektion von Saatgut mit strömungstechnisch geformtem Produktführungskanal und Gasgebläse, hermetisch gekapseltem Elektronenbeschleuniger mit Elektronenaustrittsfenster und Schutzgitter sowie Produktzuführung mit Vibrationsfördereinrichtung, Rotationseinrichtung für die Partikel, Produktausschleusung und eine integrierte Röntgenschutzeinrichtung,

Fig. 2: einen Schnitt durch einen Teil der Einrichtung gemäß Fig. 1 im Bereich des Produktführungskanals mit Gasführung und Bypass-Strömung zur Freihaltung des Gitters im Produktführungskanal und Kühlung des Elektronenaustrittsfensters,

Fig. 3: einen Schnitt durch einen Teil der Einrichtung gemäß Fig. 1 im Bereich der Rotationseinrichtung für die Partikel,

Fig. 4: einen Schnitt durch einen Teil der Einrichtung gemäß Fig. 1 im Bereich der Messeinrichtung zu Ermittlung der Elektronenstromdichte.

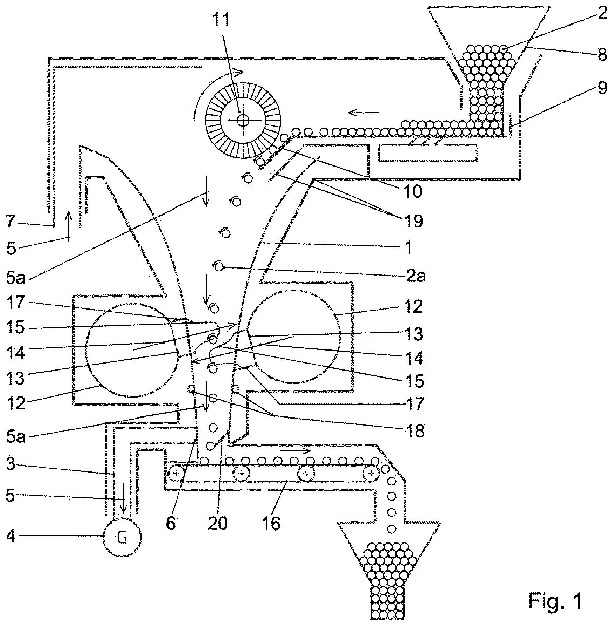

In Fig. 1 ist der prinzipielle Aufbau der beispielhaften Einrichtung mit Produktführungskanal 1 dargestellt, der in Fallrichtung der Saatgut-Partikel 2a eine kontinuierlich abnehmende Spaltbreite aufweist. Im unteren Bereich ist der Produktführungskanal 1 über eine Saugleitung 3 mit einem Gasgebläse 4 verbunden, das einen kontinuierlichen Gasstrom 5 nach außen fördert. Durch dieses kontinuierliche Absaugen wird im Inneren der Behandlungskammer ein Unterdruck erzeugt. Zum Schutz vor dem Absaugen von Saatgut-Partikeln 2a ist am Eingang der Saugleitung 3 ein Gitter 6 angeordnet. Im oberen Bereich des Produktführungskanals 1 befindet sich ein Einströmstutzen 7 zur Zuführung des Gasstromes 5. Als Prozessgas wird Luft, Kohlenstoffdioxid oder Stickstoff verwendet. Die in Fallrichtung definiert abnehmende Spaltbreite des Produktführungskanals 1 bewirkt eine kontinuierliche Beschleunigung des Gases 5a innerhalb des Produktführungskanals 1 in gleichem Maße wie die durch Gravitation auf die Saatgut-Partikel 2a wirkende Beschleunigung.

Das Saatgut 2 wird über einen Pufferbehälter 8 dem Prozess zugeführt. Am Auslauf des Pufferbehälters 8 befindet sich eine Dosiereinrichtung (nicht dargestellt), die den Volumenstrom des Saatgutes 2 auf ein definiertes Maß begrenzt. Das Saatgut 2 gelangt auf eine Vibrationsfördereinrichtung 9, die eine kontinuierliche Zuführung und Vorvereinzelung bewirkt. Die Vibrationsfördereinrichtung 9 besitzt innerhalb des Produktführungskanals 1 ein abgewinkeltes Segment 10 mit einer angerauten Oberfläche zur Erhöhung der Reibung. Eine rotierende Bürstenwalze 11 bildet mit dem Segment 10 einen einstellbaren Spalt, der dem Durchmesser der Saatgut-Partikel 2a entspricht. Durch die Rotationsbewegung der Bürstenwalze 11 erhalten die auf dem Segment 10 abrollenden Partikel des Saatgutes 2 einen Rotationsimpuls, bevor sie in den Fall mit gleichmäßiger Beschleunigung durch Gravitation übergehen.

Auf beiden Seiten des Produktführungskanals 1 sind Elektronenbeschleuniger 12 angeordnet. Der durch die Elektronenbeschleuniger 12 erzeugte flächige Elektronenstrahl 14 gelangt durch das Elektronenaustrittsfenster 13 in den Produktführungskanal 1 und bewegt sich unter Ausbildung eines in Darstellungsebene gaussförmigen Intensitätsprofiles 15 mit einer Halbwertsbreite von etwa 30 mm in Richtung der Saatgut-Partikel 2a. In Z-Achse, senkrecht zur Darstellungsebene, besitzt der Elektronenstrahl 14 eine Dimension von etwa 1000 mm. Der Elektronenstrahl 14 wird bei seiner Ausbreitung an Luft weiter gestreut und wirkt diffus und allseitig auf die im Gasstrom 5a geführten, fallenden und rotierenden Saatgut-Partikel 2a ein. Nach Einwirkung des Elektronenstrahles 14 verlässt das Saatgut über ein direkt darunter angeordnetes Hochgeschwindigkeitsband 16 innerhalb von weniger als einer Sekunde den Produktführungskanal 1, wodurch die Einwirkung von Röntgenstrahlung auf ein Minimum reduziert ist.

Die Elektronenbeschleuniger 12 sind um ihre Längsachse (z-Achse) gedreht angeordnet, so dass die Hauptbewegungsrichtung des Elektronenstrahles 14 nicht rechtwinklig zur Bewegungsrichtung der Saatgut-Partikel 2a steht. Dadurch wird der jeweils gegenüberliegende Elektronenbeschleuniger 12 nicht getroffen und auch hohe Elektronenenergien sowie Elektronenströme können das gegenüberliegende Elektronenaustrittsfenster nicht schädigen. Neben den Elektronenaustrittsfenstern 13 sind am Produktführungskanal 1 Messeinrichtungen 17 in Form von Molybdänplatten zur Aufnahme eines von der Elektronenstromverteilung abhängigen Messsignals angeordnet.

Ein optisches Messsystem 18 ermöglicht die Messung der Dichte der Saatgut-Partikel 2a und die Feststellung von Produktstaus.

Das Gesamtsystem ist mit einer Röntgenschutzverkleidung 19 ausgestattet, die den Austritt von Röntgenstrahlung in die Umwelt verhindert. Durch das Design mit Integration der Vibrationsfördereinrichtung 9 und des Hochgeschwindigkeitsbandes 16 und innenliegenden Barrieren 19 ist die Expositionszeit der Saatgut-Partikel 2a im Bereich der Röntgenstrahlung auf ein Minimum reduziert.

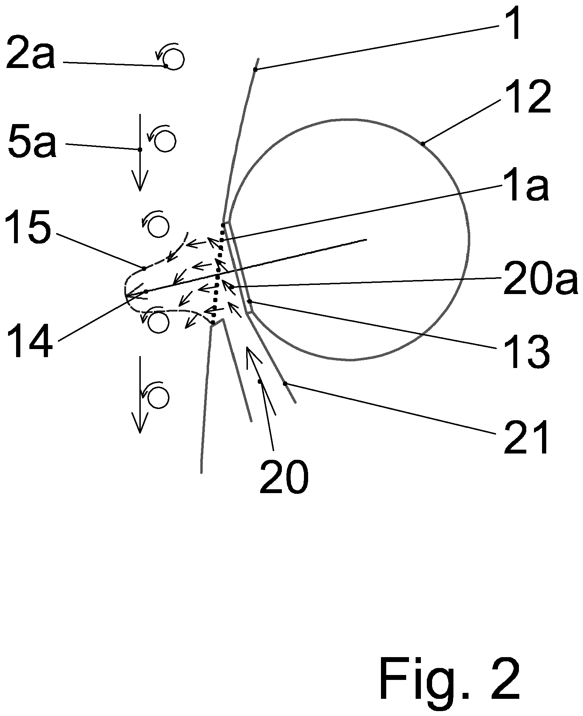

In Fig. 2 ist ein Schnitt durch einen Teil der Einrichtung gemäß Fig. 1 mit der rechten Seite des Produktführungskanals 1 und einem Elektronenbeschleuniger 12 vergrößert, jedoch nur schematisch, dargestellt. Dieser Bereich ist die Wirkzone des Elektronenstrahles, in dem die eigentliche Desinfektion stattfindet. Der Elektronenstrahl 14 mit der gaussförmigen Intensitätsverteilung 15 wird durch den perforierten Bereich 1a in den Produktführungskanal 1 geführt und wirkt allseitig auf die rotierenden Saatgut-Partikel 2a ein. Der durch die Gasströmung 5a erzeugte statische Unterdruck bewirkt eine Gasströmung 20, die von außen durch den perforierten Bereich 1a in den Produktführungskanal 1 hinein gerichtet ist und diesen von Verunreinigungen und Partikeln freihält. Ein Strömungskanal 21 bewirkt das gerichtete Nachströmen von Luft mit parallel zum Elektronenaustrittsfenster 13 ausgeprägter Vorzugsrichtung und dessen Kühlung durch Konvektion.

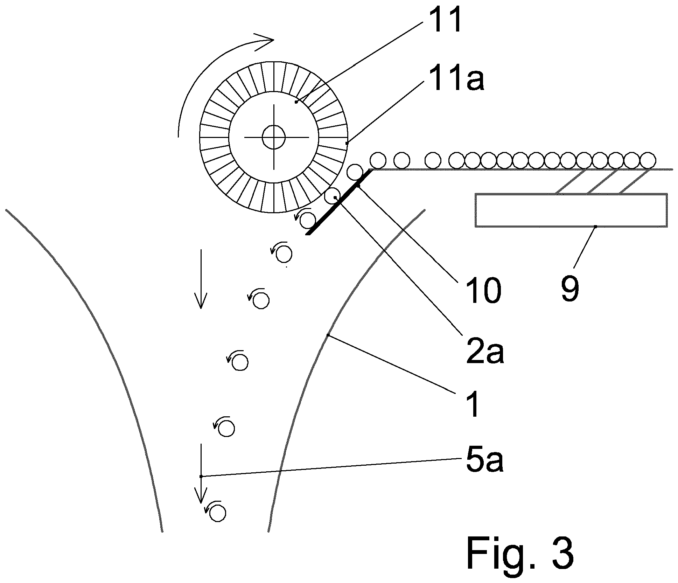

Fig. 3 zeigt einen vergrößerten schematischen Schnitt durch einen Teil der Einrichtung gemäß Fig. 1 mit der Rotationseinrichtung für Saatgut-Partikel 2a. Die rotierende Bürstenwalze 11 erfasst die Saatgut-Partikel 2a und versetzt sie auf der rauen Oberfläche des Segmentes 10 der Vibrationsfördereinrichtung 9 in eine Rotationsbewegung. Korngrößenunterschiede werden durch die elastischen Borsten 11a kompensiert, so dass nach Verlassen der Rotationseinrichtung alle Saatgut-Partikel 2a rotieren.

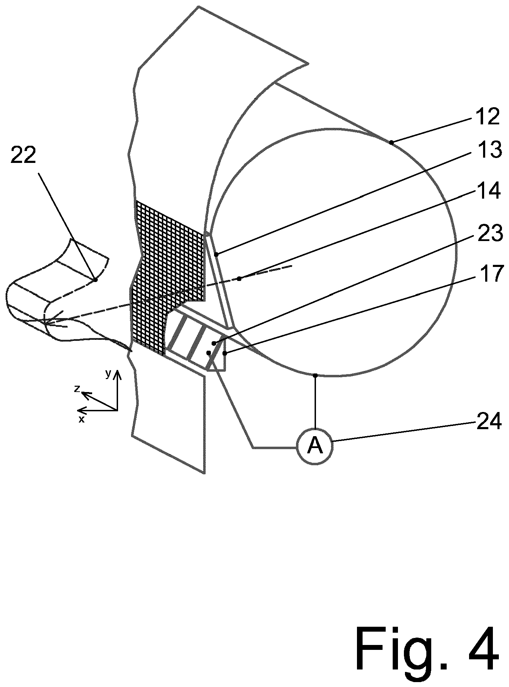

In der Vorrichtung, die in Fig. 4 schematisch dargestellt ist, ist an der Seite des Elektronenaustrittsfensters 13 eine Messeinrichtung 17 zur Ermittlung der in z-Achse zur Darstellungsebene vorliegenden Elektronenstromdichteverteilung 22 angeordnet. Sie besteht aus mehreren im Bereich der Randelektronen des Elektronenstrahles positionierten temperaturbeständigen Molybdänblechen 23. Die Molybdänbleche sind durch elektrische Leiter mit dem Potential des Gehäuses des Elektronenbeschleunigers 12 gekoppelt. Eine in Reihe geschaltete Messeinrichtung 24 dient zur Erfassung des abfließenden Elektronenstromes.

**Bezugszeichenliste**

| | |
|---|---|
| 1 | Produktführungskanal |
| 1 a | perforierter Bereich des Produktführungskanals |
| 2 | Saatgut (Produkt) |
| 2a | Saatgut-Partikel |
| 3 | Saugleitung |
| 4 | Gasgebläse |
| 5 | Gasstrom |
| 5a | Gasstrom in Fallrichtung |
| 6 | Gitter |
| 7 | Einströmstutzen |
| 8 | Pufferbehälter |
| 9 | Vibrationsfördereinrichtung |
| 10 | abgewinkeltes Segment |
| 11 | Bürstenwalze |
| 11 a | elastische Borsten der Bürstenwalze |
| 12 | Elektronenbeschleuniger |
| 13 | Elektronenaustrittsfenster |
| 14 | Elektronenstrahl |
| 15 | Intensitätsverteilung des Elektronenstroms |
| 16 | Hochgeschwindigkeitsband |
| 17 | Messeinrichtung für Elektronenstromverteilung |
| 18 | optisches Messsystem |
| 19 | Röntgenschutzverkleidung |
| 20 | zulaufende Gasströmung |
| 20a | zulaufende Gasströmung zur Kühlung des Elektronenaustrittsfensters |
| 21 | Strömungskanal |
| 22 | Elektronenstromdichteverteilung |
| 23 | Molybdänblech |
| 24 | Messeinrichtung |

**Patentansprüche**

1. Verfahren zur Behandlung rieselfähiger Produkte aus vereinzelbaren Partikeln, vorzugsweise Saatgut, mit beschleunigten Elektronen, bei dem ein transparent vereinzelter Produktstrom in einem Produktführungskanal durch das von mindestens einem Elektronenbeschleuniger erzeugte Elektronenfeld bei Unter- oder Überdruck unter Nutzung der Schwerkraft geführt wird, **dadurch gekennzeichnet, dass** der Produktführungskanal einen sich in Bewegungsrichtung der Partikel verringernden Querschnitt aufweist und die Führung des Produktstromes mittels eines beschleunigten Gasstromes erfolgt, dessen Bewegung in Betrag und Richtung der beschleunigten Bewegung entspricht, die die in ihm fallenden Partikel aufgrund der Erdbeschleunigung ausführen.

2. Verfahren nach Anspruch 1, **dadurch gekennzeichnet, dass** die Beschleunigung des Gasstromes durch Absaugung am Auslauf und/oder durch Einblasen am Einlauf des Produktführungskanals erfolgt, der sich in Transportrichtung verengt.

3. Verfahren nach Anspruch 1 oder 2, **dadurch gekennzeichnet, dass** den Partikeln vor oder beim Eintritt in den Produktführungskanal durch Abrollen auf einer schiefen Ebene oder durch kurzzeitigen gleichzeitigen Kontakt mit mindestens einem feststehenden und einem rotierenden Bauteiles oder durch gleichzeitigen Kontakt mit unterschiedlich schnell rotierenden Bauteilen eine Rotationsbewegung aufgeprägt wird.

**4.** Verfahren nach einem der Ansprüche 1 bis 3, **dadurch gekennzeichnet, dass** zur Reibungsminderung zumindest in Teilbereichen des Produktführungskanals ein Gasstrom mit einer 45 ° bis 90 ° zur Bewegungsrichtung des Produktstromes gerichteten Bewegungskomponente in den Produktstrom eingebracht wird.

**5.** Verfahren nach einem der Ansprüche 1 bis 4, **dadurch gekennzeichnet, dass** bei der Behandlung des rieselfähigen Produktes mit beschleunigten Elektronen ein Prozessgas zugeführt wird.

**6.** Verfahren nach Anspruch 5, **dadurch gekennzeichnet, dass** als Prozessgas Luft oder Stickstoff oder Kohlendioxid verwendet wird.

**7.** Verfahren nacheinem der Ansprüche 1 bis 6, **dadurch gekennzeichnet, dass** das Prozessgas in einem Kreislauf geführt und/oder einer Entstaubung unterzogen wird.

**8.** Verfahren nach einem der Ansprüche 1 bis 7, **dadurch gekennzeichnet, dass** der Produktführungskanal einen rechteckigen nach dem Zusammenhang $s = k * \dfrac{1}{\sqrt{h}}$ sich verengenden Querschnitt aufweist, wobei s die Spaltbreite zwischen zwei gegenüberliegenden Seiten des Produktführungskanals, h der zurückgelegte Fallweg der Partikel und k eine Konstante ist, die vorzugsweise einen Wert zwischen 170 $mm^{3/2}$ und 250 $mm^{3/2}$ aufweist.

**9.** Vorrichtung zur Desinfektion eines Produktstromes rieselfähiger Partikel, vorzugsweise Saatgut, mit beschleunigten Elektronen, aufweisend

• eine mit Gas gefüllte Prozesskammer mit einem vertikalen Produktführungskanal zur Führung des Produktstromes,
• Mittel zur schleusungsfreien, vereinzelnden Zuführung der rieselfähigen Partikel zum oberen Ende des Produktführungskanals,
• mindestens einen Elektronenbeschleuniger mit Elektronenaustrittsfenster und den zugehörigen Hochspannungs- und Steuersystemen,

◦ der seitlich an dem Produktführungskanal angeordnet ist
◦ dessen Elektronenstrahlen auf den durch den Produktführungskanal geführte Produktstrom treffen und so eine Behandlungszone ausbilden, wobei
◦ die Emissionsbreite des Elektronenbeschleunigers mindestens der Breite des Produktstromes entspricht

• Mittel zur schleusungsfreien Abführung des Produktstromes,
• Mittel zur Erzeugung eines Unter- oder Überdrucks in der Prozesskammer **dadurch gekennzeichnet, dass**
• der Produktkanal einen in Bewegungsrichtung der Partikel sich kontinuierlich verringernden Querschnitt aufweist,
• die Mittel zur Erzeugung eines Unter- oder Überdrucks in der Prozesskammer und die Ausbildung des Partikelführungskanals einen konstanten Volumenstrom des Gases im Produktführungskanal hervorrufen, der eine zur Bewegung der Partikel in Betrag und Richtung gleiche beschleunigte Bewegung des Gases erzeugt.

**10.** Vorrichtung nach Anspruch 9, **dadurch gekennzeichnet, dass** der Produktführungskanal einen rechteckigen Querschnitt aufweist und in seinem Verlauf auf mindestens einer der zweilängeren gegenüberliegenden Seiten so gewölbt ist, dass sich sein Querschnitt in Transportrichtung verringert.

**11.** Vorrichtung nach Anspruch 9, **dadurch gekennzeichnet, dass** vor bzw. über der Behandlungszone eine Vorrichtung zur Erzeugung einer Rotationsbewegung der Partikel des Produktstromes angeordnet ist.

**12.** Vorrichtung nach Anspruch 10, **dadurch gekennzeichnet, dass** die Spaltbreite s zwischen den zwei längeren gegenüberliegenden Seiten des Produktführungskanals in Bezug auf die Fallhöhe h der Partikel dem Zusammenhang $s = k * \dfrac{1}{\sqrt{h}}$ folgt, wobei s die Spaltbreite zwischen zwei gegenüberliegenden Seiten des Produktführungskanals, h der zurückgelegte Fallweg der Partikel und k eine Konstante ist, die vorzugsweise einen Wert zwischen 170 $mm^{3/2}$ und 250 $mm^{3/2}$ aufweist.

**13.** Vorrichtung nacheinem der Ansprüche 9 bis 12, **dadurch gekennzeichnet, dass** der Elektronenbeschleuniger ein Flächenstrahlerzeuger mit hermetisch abgeschlossenem evakuiertem Beschleunigerraum ist, für dessen Betrieb keine Vakuumpumpen erforderlich sind.

**14.** Vorrichtung nacheinem der Ansprüche 9 bis 13, **dadurch gekennzeichnet, dass** der Produktführungskanal im Bereich des Elektronenaustrittsfensters eine Perforation aufweist, die zur Fernhaltung von Staub eine Gasströmung in Richtung Produktstrom sowie den Eintritt der Elektronen in den Produktführungskanal und deren Einwirkung auf das Produkt ermöglicht.

**15.** Vorrichtung nacheinem der Ansprüche 9 bis 14, **dadurch gekennzeichnet, dass** die Mittel zur Erzeugung des Unter- oder Überdrucks Gasfördereinrichtungen sind.

**16.** Vorrichtung nach Anspruch 15, **dadurch gekennzeichnet, dass** die Mittel zur Erzeugung des Unter- oder Überdrucks das Gas der Behandlungskammer im Kreislauf fördern und in dem Förderkreis wahlweise eine Entstaubungsvorrichtung angeordnet ist.

**17.** Vorrichtung nacheinem der Ansprüche 9 bis 14, **dadurch gekennzeichnet, dass** zwischen Produktführungskanal und Elektronenaustrittsfenster und/oder im Produktführungskanal eine Messeinrichtung zur Erfassung der Elektronenstromdichte angeordnet ist.

**18.** Vorrichtung nach Anspruch 17, **dadurch gekennzeichnet, dass** die Messeinrichtung aus mindestens zwei elektrisch isoliert angeordneten metallischen Segmenten besteht, die durch elektrische Leiter mit dem Erdpotenzial gekoppelt sind und dem elektrischen Leiter eine Messeinrichtung zur Erfassung des abfließenden Elektronenstromes zwischengeschaltet ist, um ein von der Elektronenstromdichte abhängiges Signal zu erzeugen.

**19.** Vorrichtung nacheinem der Ansprüche 9 bis 17, **dadurch gekennzeichnet, dass** bei dem Durchtrittsbereich der Elektronen im Produktführungskanal eine Messeinrichtung zur Erfassung von Produktstaus angeordnet ist.

**20.** Vorrichtung nach Anspruch 19, **dadurch gekennzeichnet, dass** die Messeinrichtung aus mindestens zwei zueinander und zur Wandung des Produktführungskanals elektrisch isolierten metallischen Segmenten besteht, die mittels einer zwischen den Segmenten herrschenden Prüfspannung ein von der Partikelstromdichte abhängiges Signal liefern oder dass die Messeinrichtung ein optisches System ist, das ein von der Partikelstromdichte abhängiges Signal liefert.

**21.** Vorrichtung nach einem der Ansprüche 9 bis 20, **dadurch gekennzeichnet, dass** zur Produktzuführung eine mit einer Röntgenschutzeinrichtung ausgestattete Vibrationsfördereinrichtung angeordnet und/oder dass zur Produktabführung ein mit einer Röntgenschutzeinrichtung ausgestattete Fördereinrichtung angeordnet ist.

**22.** Vorrichtung nach einem der Ansprüche 9 bis 20, **dadurch gekennzeichnet, dass** dem nach außen geführten Gasstrom von dem Ausblasen in die Umwelt ein Ozonkatalysator vorgeschaltet ist.

**Claims**

**1.** A method for the treatment of pourable products comprising separable particles, preferably seeds, with accelerated electrons, in which a transparently isolated product flow is guided using gravity in a product guiding channel through the electron field generated by at least one electron accelerator at negative or excess pressure, **characterized in that** the product guiding channel has a decreasing cross section in the movement direction of the particles and the product flow is guided by means of an accelerated gas flow, the movement whereof corresponds in magnitude and direction to the accelerated movement which the falling particles execute in said gas flow on account of gravitational acceleration.

**2.** The method according to claim 1, **characterized in that** the acceleration of the gas flow takes place through suction at the outlet and/or through injection at the inlet of the product guiding channel which narrows in the transport direction.

**3.** The method according to claim 1 or 2, **characterized in that** the particles are imparted with a rotational movement before or during entry into the product guiding channel, by unrolling on an inclined plane or through short-term simultaneous contact with at least one fixed and one rotating component or through simultaneous contact with components rotating at different speeds.

4. The method according to one of claims 1 to 3, **characterized in that** in order to reduce friction at least in partial areas of the product guiding channel, a gas flow with a movement component directed at 45° to 90° in respect of the movement direction of the product flow is introduced into the product flow.

5. The method according to one of claims 1 to 4, **characterized in that** during the treatment of the pourable product using accelerated electrons a process gas is supplied.

6. The method according to claim 5, **characterized in that** air or nitrogen or carbon dioxide is used as the process gas.

7. The method according to one of claims 1 to 6, **characterized in that** the process gas is conducted in a circuit and/or undergoes dedusting.

8. The method according to one of claims 1 to 7, **characterized in that** the produce guiding channel has a narrowing rectangular cross section according to the correlation $s = k * \dfrac{1}{\sqrt{h}}$ where s is the gap width between two opposite sides of the product guiding channel, h is the drop path travelled by the particle and k is a constant which preferably has a value of between 170 $mm^{3/2}$ and 250 $mm^{3/2}$.

9. A device for disinfecting a product flow of pourable particles, preferably seed, with accelerated electrons, having

   • a process chamber filled with gas with a vertical product guiding channel for guiding the product flow,
   • means for airlock-free, isolating guiding of pourable particles to the upper end of the product guiding channel,
   • at least one electron accelerator with an electron exit window and the associated high voltage and control systems,

      ◦ which is arranged laterally on the product guiding channel,
      ◦ the electron beams of which hit the product flow guided through the product guiding channel and thus form a treatment zone, wherein
      ◦ the emission width of the electron accelerator corresponds at least to the width of the product flow,

   • means for the airlock-free removal of the product flow,
   • means for generating a negative or excess pressure in the process chamber, **characterized in that**
   • the product channel has a cross section constantly narrowing in the movement direction of the particles,
   • the means for generating a low pressure or excess pressure in the process chamber and the design of the particle guiding channel produce a constant volume flow of gas in the product guiding channel which generates an accelerated movement of the gas identical to the movement of the particles in magnitude and direction.

10. The device according to claim 9, **characterized in that** the product guiding channel has a rectangular cross section and is curved over its course on at least one of the two longer opposite sides such that its cross section decreases in the transport direction.

11. The device according to claim 9, **characterized in that** a device for generating a rotational movement of the particles of the product flow is arranged before or over the treatment zone.

12. The device according to claim 10, **characterized in that** the gap width s between the two longer opposite sides of the product guiding channel follows the correlation $s = k * \dfrac{1}{\sqrt{h}}$ in relation to the drop height h of the particles, where s is the gap width between two opposite sides of the product guiding channel, h is the drop path travelled by the particle and k is a constant that preferably has a value between 170 $mm^{3/2}$ and 250 $mm^{3/2}$.

13. The device according to one of claims 9 to 12, **characterized in that** the electron accelerator is a flat beam generator with a hermetically-sealed, evacuated acceleration chamber, for the operation of which no vacuum pumps are necessary.

**14.** The device according to one of claims 9 to 13, **characterized in that** the product guiding channel has a perforation in the area of the electron outlet window, which perforation allows a gas flow in the direction of the product flow to keep dust away and also the entry of electrons into the product guiding channel and the influence thereof on the product.

**15.** The device according to one of claims 9 to 14, **characterized in that** the means for generating the negative pressure or excess pressure are gas conveying devices.

**16.** The device according to claim 15, **characterized in that** the means for generating the negative pressure or excess pressure convey the gas of the treatment chamber in a circuit and a dedusting device may optionally be arranged in the conveying circuit.

**17.** The device according to one of claims 9 to 14, **characterized in that** a measuring device for detecting the electron beam density is arranged between the product guiding channel and the electron outlet window and/or in the product guiding channel.

**18.** The device according to claim 17, **characterized in that** the measuring device comprises at least two metal segments arranged in an electrically insulated manner, which segments are coupled to the ground potential via electrical conductors, and a measuring device for detecting the outgoing electron flow is interconnected to the electrical conductor, in order to generate a signal depending on the electron flow density.

**19.** The device according to one of claims 9 to 17, **characterized in that** a measuring device for detecting the product status is arranged at the passage area of the electrons in the product guiding channel.

**20.** The device according to claim 19, **characterized in that** the measuring device comprises at least two metal segments electrically insulated from each other and from the walls of the product guiding channel, which segments deliver a signal depending on the particle flow density by means of a test voltage prevailing between the segments or that the measuring device is an optical system which supplies a signal dependent on the particle flow density.

**21.** The device according to one of claims 9 to 20, **characterized in that** a vibration conveying device equipped with an X-ray protection device is arranged for supplying the product and/or that a conveying device equipped with an X-ray protection device is arranged for removing the product.

**22.** The device according to one of claims 9 to 20, **characterized in that** an ozone catalytic converter is connected upstream of the gas flow guided outward into the environment by the exhaust.

## Revendications

**1.** Procédé de traitement de produits aptes à s'écouler ou granuleux composés de particules individualisables, de préférence de semences, avec des électrons accélérés, selon lequel un flux de produit individualisé de façon transparente est guidé dans un canal de guidage de produit à travers le champ d'électrons produit par au moins un accélérateur d'électrons sous dépression ou surpression en utilisant la force de gravité, **caractérisé en ce que** le canal de guidage de produit présente une section transversale diminuant dans la direction de mouvement des particules et que le guidage du flux de produit s'effectue au moyen d'un flux de gaz accéléré dont le mouvement correspond, en valeur et en direction, au mouvement accéléré que décrivent les particules qui y tombent sous l'effet de la force de gravité.

**2.** Procédé selon la revendication 1, **caractérisé en ce que** l'accélération du flux de gaz s'effectue par aspiration à la sortie et/ou par soufflage à l'entrée du canal de guidage de produit qui se rétrécit dans la direction de transport.

**3.** Procédé selon l'une des revendications 1 ou 2, **caractérisé en ce qu'**avant ou à leur entrée dans le canal de guidage de produit, un mouvement de rotation est imprimé aux particules par roulement sur un plan incliné ou par contact simultané de courte durée avec au moins un élément fixe et un élément en rotation ou par contact simultané avec des éléments en rotation à des vitesses différentes.

**4.** Procédé selon l'une des revendications 1 à 3, **caractérisé en ce que** pour diminuer le frottement au moins dans des zones partielles du canal de guidage de produit, un flux de gaz avec une composante de mouvement faisant un angle de 45° à 90° avec la direction de mouvement du flux de produit est introduit dans le flux de produit.

5. Procédé selon l'une des revendications 1 à 4, **caractérisé en ce qu'**un gaz de procédé est amené lors du traitement du produit apte à s'écouler avec des électrons accélérés.

6. Procédé selon la revendication 5, **caractérisé en ce que** de l'air, de l'azote ou du dioxyde de carbone est utilisé comme gaz de procédé.

7. Procédé selon l'une des revendications 1 à 6, **caractérisé en ce que** le gaz de procédé est guidé en circuit fermé et/ou soumis à un dépoussiérage.

8. Procédé selon l'une des revendications 1 à 7, **caractérisé en ce que** le canal de guidage de produit présente une section transversale rectangulaire qui se rétrécit selon la relation s = k * 1/racine carrée(h), dans laquelle s est la largeur de passage entre deux côtés opposés du canal de guidage de produit, h la hauteur de chute des particules et k une constante qui présente de préférence une valeur comprise entre 170 mm$^{3/2}$ et 250 mm$^{3/2}$.

9. Dispositif de désinfection d'un flux de produit de particules aptes à s'écouler, de préférence de semences, avec des électrons accélérés, présentant

   • une chambre de traitement remplie de gaz comportant un canal de guidage de produit vertical pour le guidage du flux de produit,
   • des moyens pour l'amenée individualisée, sans éclusage, des particules aptes à s'écouler à l'extrémité supérieure du canal de guidage de produit,
   • au moins un accélérateur d'électrons avec fenêtre de sortie d'électrons et les systèmes à haute tension et de commande associés,

      - qui est disposé latéralement au canal de guidage de produit
      - dont les faisceaux d'électrons rencontrent le flux de produit guidé à travers le canal de guidage de produit et forment ainsi une zone de traitement,
      - la largeur d'émission de l'accélérateur d'électrons correspondant au moins à la largeur du flux de produit

   • des moyens pour l'évacuation sans éclusage du flux de produit,
   • des moyens pour produire une dépression ou une surpression dans la chambre de traitement,

   **caractérisé en ce que**

   • le canal de produit présente une section transversale diminuant de façon continue dans la direction de mouvement des particules,
   • les moyens pour produire une dépression ou une surpression dans la chambre de traitement et la configuration du canal de guidage de particules provoquent un débit volumique constant du gaz dans le canal de guidage de produit, qui produit un mouvement accéléré du gaz identique en valeur et en direction au mouvement des particules.

10. Dispositif selon la revendication 9, **caractérisé en ce que** le canal de guidage de produit présente une section transversale rectangulaire et est, dans son étendue, cintré sur au moins un des deux grands côtés opposés de telle sorte que sa section transversale diminue dans la direction de transport.

11. Dispositif selon la revendication 9, **caractérisé en ce qu'**un dispositif pour produire un mouvement de rotation des particules du flux de produit est disposé avant ou au-dessus de la zone de traitement.

12. Dispositif selon la revendication 10, **caractérisé en ce que** la largeur de passage s entre les deux grands côtés opposés du canal de guidage de produit par rapport à la hauteur de chute h des particules suit la relation s = k * 1/racine carrée(h), dans laquelle s est la largeur de passage entre deux côtés opposés du canal de guidage de produit, h la hauteur de chute des particules et k une constante qui présente de préférence une valeur comprise entre 170 mm$^{3/2}$ et 250 mm$^{3/2}$.

13. Dispositif selon l'une des revendications 9 à 12, **caractérisé en ce que** l'accélérateur d'électrons est un générateur de faisceau en nappe avec chambre d'accélération mise au vide et fermée hermétiquement, pour le fonctionnement duquel aucune pompe à vide n'est nécessaire.

**14.** Dispositif selon l'une des revendications 9 à 13, **caractérisé en ce que** le canal de guidage de produit présente dans la zone de la fenêtre de sortie d'électrons une perforation qui permet un écoulement de gaz dans la direction du flux de produit pour éviter la poussière ainsi que l'entrée des électrons dans le canal de guidage de produit et leur action sur le produit.

**15.** Dispositif selon l'une des revendications 9 à 14, **caractérisé en ce que** les moyens pour produire la dépression ou la surpression sont des dispositifs de transport de gaz.

**16.** Dispositif selon la revendication 15, **caractérisé en ce que** les moyens pour produire la dépression ou la surpression transportent le gaz de la chambre de traitement en circuit fermé et qu'un dispositif de dépoussiérage est disposé au choix dans le circuit de transport.

**17.** Dispositif selon l'une des revendications 9 à 14, **caractérisé en ce qu'**un dispositif de mesure pour détecter la densité du flux d'électrons est disposé entre le canal de guidage de produit et la fenêtre de sortie d'électrons et/ou dans le canal de guidage de produit.

**18.** Dispositif selon la revendication 17, **caractérisé en ce que** le dispositif de mesure est constitué d'au moins deux segments métalliques isolés électriquement qui sont couplés au potentiel de la terre par des conducteurs électriques et un dispositif de mesure pour détecter le flux d'électrons qui s'écoule est intercalé entre les conducteurs électriques afin de produire un signal dépendant de la densité du flux d'électrons.

**19.** Dispositif selon l'une des revendications 9 à 17, **caractérisé en ce qu'**un dispositif de mesure pour détecter des bourrages de produit est disposé près de la zone de passage des électrons dans le canal de guidage de produit.

**20.** Dispositif selon la revendication 19, **caractérisé en ce que** le dispositif de mesure est constitué d'au moins deux segments métalliques isolés électriquement l'un par rapport à l'autre et par rapport à la paroi du canal de guidage de produit, qui fournissent un signal dépendant de la densité du flux de particules au moyen d'une tension de contrôle régnant entre les segments, ou que le dispositif de mesure est un système optique qui fournit un signal dépendant de la densité du flux de particules.

**21.** Dispositif selon l'une des revendications 9 à 20, **caractérisé en ce qu'**un dispositif de transport à vibrations équipé d'un dispositif de protection contre le rayonnement X est disposé pour l'amenée du produit et/ou qu'un dispositif de transport équipé d'un dispositif de protection contre le rayonnement X est disposé pour l'évacuation du produit.

**22.** Dispositif selon l'une des revendications 9 à 20, **caractérisé en ce qu'**un catalyseur d'ozone est disposé en amont dans le flux de gaz guidé vers l'extérieur avant son rejet dans l'environnement.

Fig. 1

Fig. 2

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DD 291704
- DD 291705
- DD 291677
- EP 0705531 A
- DE 4434767
- EP 1080623 A